# EUROPÄISCHE PATENTANMELDUNG

(11) **EP 0 207 358 A2**
(43) Veröffentlichungstag der Anmeldung: **07.01.1987**
(21) Anmeldenummer: 86108232.9
(22) Anmeldetag: 16.06.1986
(51) Int. Cl.: A23K 1/16, C07C 127/19, C07C 149/437, C07C 157/09, C07D 333/00, C07D 209/20, C07D 233/64

(54) **Leistungsfördernde Mittel**

(30) Priorität: 27.06.1985 DE 3522938
(71) Anmelder: BAYER AG, 51368 Leverkusen (DE)
(72) Erfinder: Hallenbach, Werner, Dr., D-4018 Langenfeld (DE); Lindel, Hans, Dr., D-5090 Leverkusen 1 (DE); Berschauer, Friedrich, Dr., D-5600 Wuppertal 1 (DE); Scheer, Martin, Dr., D-5600 Wuppertal 1 (DE); de Jong, Anno, Dr., D-5600 Wuppertal 1 (DE)

(57) **Zusammenfassung**

Die vorliegende Erfindung betrifft die Verwendung substituierter Harnstoffe und isoharnstoffe der Formeln la und Ib 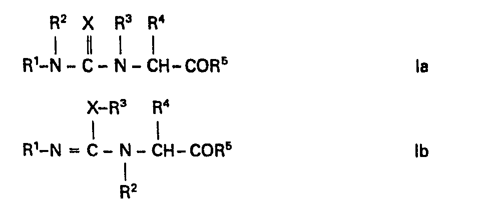 in welchen
R¹-R⁵ und X die in der Beschreibung angegebene Bedeutung haben,

als leistungsfördernde Mittel für Tiere, neue substituierte Harnstoffe und Isoharnstoffe sowie Verfahren zu ihrer Herstellung.

## Beschreibung

Die vorliegende Erfindung betrifft die Verwendung von teilweise bekannten substituierten Harnstoffen und Isoharnstoffen als leistungsfördernde Mittel für Tiere, neue substituierte Harnstoffe und Verfahren zu ihrer Herstellung.

Substituierte Harnstoffe und ihre Verwendung als Herbizide sind bereits bekannt geworden. (DE-OS 3 236 626). Monosubstituierte Harnstoffe und ihre Verwendung als Futterzusatzmittel für Geflügel und Nicht-Wiederkäuer sind bereits bekannt geworden (DE-OS 1 807 604). Durch Essigsäure substituierte Harnstoffe und ihre Verwendung als Leistungsförderer bei Tieren sind bereits bekannt geworden (DE-OS 2 501 788, DE-OS 2 505 301).

Bei den bekannten Verbindungen ist entweder nichts über ihre Eignung als Leistungsförderer bei Tieren bekannt oder sie befriedigen in ihrer Wirkung nicht voll.

Die vorliegende Erfindung betrifft:
1 Die Verwendung der teilweise bekannten substituierten Harnstoffe und Isoharnstoffe der Formeln Ia und Ib 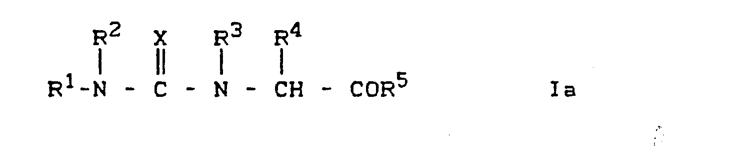 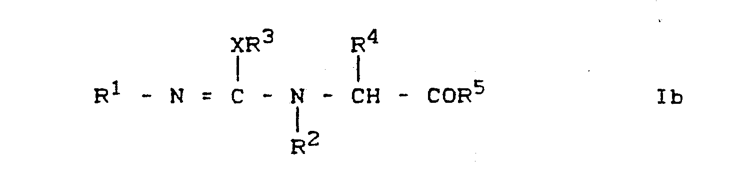 in welchen
   R¹ für Alkyl, ein- oder mehrcyclisches Cycloalkyl, Cycloalkanon, Aryl, Heteroaryl, Alkenyl, Cycloalkenyl, Cyloalkenon, die gegebenenfalls substituiert sein können steht,
   R² für Wasserstoff oder Alkyl steht,
   R³ für Wasserstoff oder Alkyl steht,
   R⁴ für Alkyl steht, das gegebenenfalls substituiert sein kann,
   R³ und R⁴ können gemeinsam mit den Atomen, an die sie gebunden sind, einen gegebenenfalls substituierten 5-gliedrigen gesättigten Ring bilden,
   R⁵ für OH, Alkyl, Alkoxy, Aryl, Aryloxy, die gegebenenfalls substituiert sein können, Amino, -NR⁶R⁷ steht,
   R⁶ für Wasserstoff oder Alkyl steht,
   R⁷ für Wasserstoff, Alkyl, Alkenyl, Alkinyl, Aryl, Aralkyl, die gegebenenfalls substituiert sein können, steht,
   X für 0 oder S steht,

   als leistungsfördernde Mittel bei Tieren.
   Die Wirkstoffe der Formeln Ia und Ib können dabei in Form ihrer Enantiomeren sowie in Form ihrer physiologisch verträglichen Salze vorliegen.
2. Substituierte Harnstoffe der Formel Ia 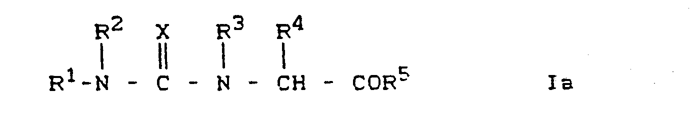 in welcher
   R^{l} für Alkyl, ein oder mehrcyclisches Cycloalkyl, Cycloalkanon, Alkenyl, Cycloalkenyl, Cycloalkenon, Naphthyl, Thiophen steht, die gegebenenfalls substituiert sein können,
   R² für Wasserstoff oder Alkyl steht,
   R³ für Wasserstoff oder Alkyl steht,
   R⁴ für substituiertes Alkyl steht,
   R⁵ für Alkyl, Alkoxy, Aryl, Aryloxy, die gegebenenfalls substituiert sein können, für Amino oder -NR⁶R⁷ steht,
   R⁶ für Wasserstoff oder Alkyl steht,
   R⁷ für Wasserstoff, Alkyl, Alkenyl, Alkinyl, Aryl, Aralkyl, die gegebenenfalls substituiert sein können, steht,

   X für O oder S steht,
   sind neu.
3. Substituierte Isoharnstoffe der Formel Ib 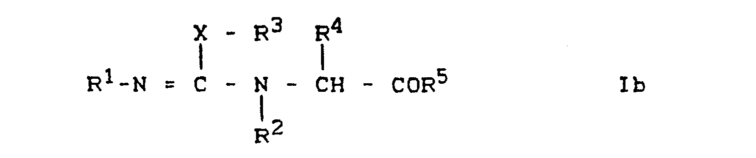 in welcher
   R⁵ für Alkyl, Alkoxy, Aryl, Aryloxy, Amino oder NR⁶R⁷ steht, die gegebenenfalls substituiert sein können,
   _{R}^{l}, _{R}², _{R}³, _{R}⁴, _{R}⁶, _{R}⁷, X die unter 1 (oben) angegebene Bedeutung haben

   sind neu.
4. Verfahren zur Herstellung substituierter Harnstoffe der Formel Ia 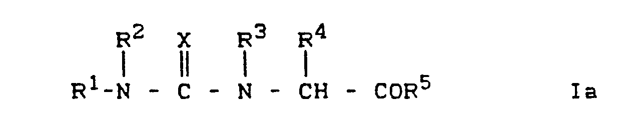 in welcher
   R¹ für Alkyl, ein oder mehrcyclisches Cycloalkyl, Cycloalkanon, Alkenyl, Cycloalkenyl, Cycloalkenon, Naphthyl, Thiophen steht, die gegebenenfalls substituiert sein können,
   R² für Wasserstoff oder Alkyl steht,
   _{R}³ für Wasserstoff oder Alkyl steht,
   _{R}⁴ für substituiertes Alkyl steht,
   R⁵ für Alkyl, Alkoxy, Aryl, Aryloxy, die gegebenenfalls substituiert sein können, für Amino oder -NR⁶R⁷ steht,
   R⁶ für Wasserstoff oder Alkyl steht,
   R⁷ für Wasserstoff, Alkyl, Alkenyl, Alkinyl, Aryl, Aralkyl, die gegebenenfalls substituiert sein können, steht,
   X für 0 oder S steht,

   dadurch gekennzeichnet, daß man
   a) für den Fall, daß R² für Wasserstoff steht, Isocyanate oder -thiocyanate der Formel II 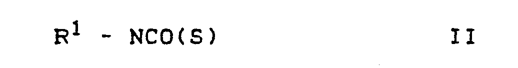 in welcher
      R¹ die oben angegebene Bedeutung hat,
         mit Aminosäurederivaten der Formel III 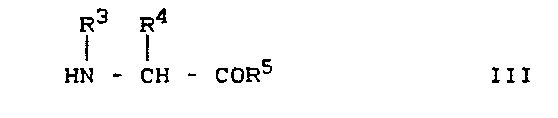 in welcher
      R³, R⁴, R⁵ die oben angegene Bedeutung haben,
         gegebenenfalls in Gegenwart von Katalysatoren und Verdünnungsmitteln umsetzt, oder
   b) für den Fall, daß R³ für Wasserstoff steht, Amine der Formel IV 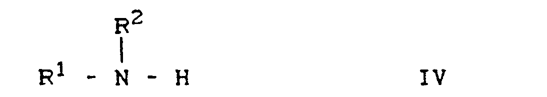 in welcher
      R¹ und R² die oben angegebene Bedeutung haben,
         mit Isocyanaten oder -thiocyanaten der Formel V 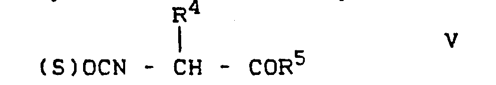 in welcher
      R⁴ und R⁵ die oben angegebene Bedeutung haben,
         gegebenenfalls in Gegenwart von Katalysatoren und Verdünnungsmitteln umsetzt.
5. Verfahren zur Herstellung von substituierten Isoharnstoffen der Formel Ib 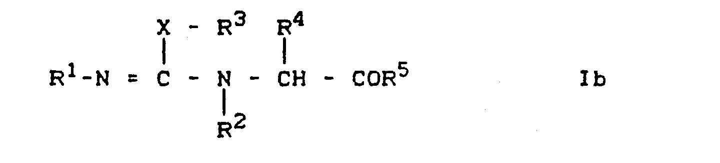 in welcher
   R⁵ für Alkyl, Alkoxy, Aryl, Aryloxy, Amino oder -NR⁶R⁷ steht, die gegebenenfalls substituiert sein können,
   R¹, _{R}², _{R}³, _{R}⁴, _{R}^{b}, R⁷, X die unter 1 (oben) angegebene Bedeutung haben,

   dadurch gekennzeichnet, daß man Imidokohlensäureesterhalogeniden der Formel VI 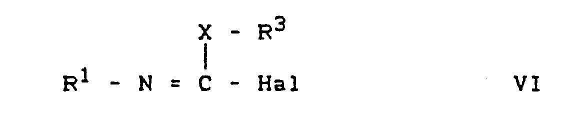 in welcher
   Hal für Halogen steht,
   X, R¹ und R³ die oben angegebene Bedeutung haben,

   mit Aminosäurederivaten der Formel VII 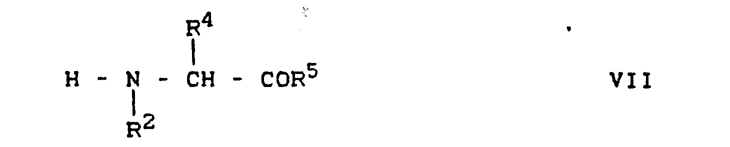 in welcher
   R², R⁴, R⁵ die oben angegebene Bedeutung haben,

   umsetzt.

Die substituierten Harnstoffe und Isoharnstoffe der Formeln Ia und Ib zeigen eine erheblich bessere leistungsfördernde Wirkung bei Tieren als die Harnstoffderivate, von denen eine solche Wirkung seither bekannt war.

Bevorzugt werden substituierte Harnstoffe und Isoharnstoffe der Formeln Ia und Ib eingesetzt in welchen
R¹ für Cₗ-₁₂-Alkyl, C₃-₁₀-Cycloalkyl, C₅₋₆-Cycloalkanon, Adamantyl, Phenyl, Naphthyl, Heteroaryl mit 5-6 Ring-atomen, wobei als Heteroatome N, 0, S enthalten sein können, insbesondere Thiophen und Hydrobenzothiophen, C₂-₁₂-Alkenyl, C₅-₈-Cycloalkenyl steht, die gegebenenfalls durch einen oder mehrere der folgenden Reste gleich oder verschieden substituiert sein können: Alkyl mit vorzugsweise 1 bis 4, insbesondere 1 oder 2 Kohlenstofatomen, wie Methyl, Ethyl, n- und i.-Propyl und n.-, i.- und t.-Butyl; ankondensiertes C₂₋₅-Alkanyl oder C₄-Alkenyli Alkoxy mit vorzugsweise 1 bis 4, insbesondere 1 oder 2 Kohlenstoffatomen, wie Methoxy, Ethoxy, n.- und i.-Propyloxy und n.-, i.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.- i.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Chlor und Brom; Cyano; Nitro; Amino; Monoalkyl-und Dialkylamino mit vorzugweise 1 bis 4, insbesondere 1 oder 2 Kohlenstoffatomen je Alkylgruppe, wie Methylamino, Methyl-ethyl-amino, n.- und i.-Propylamino und Methyl-n.-Butylamino; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder substituiert sein können.
   Substituenten an aromatischen Ringen können zusätzlich sein gegebenenfalls halogensubstituiertes Alkylendioxy insbesondere gegebenenfalls chlor- oder fluorsubstituiertes Methylen- oder Ethylendioxy.
R² für Wasserstoff oder C₁₋₄-Alkyl steht,
R³ für Wasserstoff oder C₁₋₄-Alkyl steht,
R⁴ für C₁₋₄-Alkyl steht, das gegebenenfalls durch Aryl insbesondere Phenyl, OH, SH, C₁₋₄-Alkylthio, COOH; CONH₂, COOC₁₋₄-Alkyl, Heteroaryl insbesondere Imidazolyl, Indolyl, Benzofuranyl, Benzothienyl, substituiert sein kann,
R³ und R⁴ können im Fall der Harnstoffe der Formel Ia gemeinsam mit den Atomen, an die sie gebunden sind einen gegebenenfalls durch OH substituierten 5-gliedrigen gesättigten Ring bilden.
_{R}⁵ für OH, Cₗ₋₄-Alkyl, C₁₋₄-Alkoxy, Phenyl, Phenoxy steht, die gegebenenfalls durch einen oder mehrere, gleiche oder verschiedene der folgenden Substituenten substituiert sein können: Halogen, CN, C₁₋₄-Alkyl, C₁₋₄-Alkoxy, Phenyl, Phenoxy, Phenylthio, die ihrerseits substituiert sein können, sowie für NH₂ oder _{NR}⁶_{R}⁷ steht,
R⁶ für Wasserstoff oder C₁₋₄-Alkyl steht,
R⁷ für Wasserstoff, C₁₋₄-Alkyl, C₂₋₄-Alkenyl, C₂-₄-Alkinyl, Phenyl, Naphthyl, C₁₋₂-Alkylphenyl steht, die gegebenenfalls durch Halogen, CN, C₁₋₄-Alkyl, C₁-₄-Alkoxy substituiert sein können,
X für 0 oder S steht.

Besonders bevorzugt werden Verbindungen der Formel Ia und Ib eingesetzt, in welchen
R¹ für C₁₋₄-Alkyl, C₃_₆-Cycloalkyl, mehrcyclische Cycloalkyle wie z.B. Adamantyl, ferner Phenyl, Naphthyl, Heteroaryle mit 5-6 Ring-Atomen insbesondere Thiophen, Pyrrol, Furan, die gegebenenfalls an weitere cyclische gesättigte oder ungesättigte 5- bis 7-gliedrige Ringe ankondensiert sein können, C₂₋₆-Alkenyl, Cyclopentyl, Cyclohexenyl steht, wobei diese Reste durch einen oder mehrere der folgenden Substituenten substituiert sein können:
   Alkyl mit vorzugsweise 1 bis 4, insbesondere 1 oder 2 Kohlenstoffatomen, wie Methyl, Ethyl, n- und i.-Pro pyl und n.-, i.- und t.-Butyl; ankondensierte C₂₋₄-Alkenyl oder C₄-Alkenyl; Alkoxy mit vorzugsweise 1 bis 4, insbesondere 1 oder 2 Kohlenstoffatomen, wie Methoxy, Ethoxy, n.- und i.-Propyloxy und n.-, i.-und t.-Butyloxy; Alkylthio mit vorzugsweise 1 bis 4, insbesondere 1 oder 2 Kohlenstoffatomen, wie Methyl- thio, Ethylthio, n.- und i.-Propylthio und n.- i.-und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, insbesondere Chlor; Cyano; Nitro; Amino; Monoalkyl- und Dialkylamino mit vorzugweise 1 bis 4, insbesondere 1 oder 2 Kohlenstoffatomen je Alkylgruppe, wie Methylamino, Methyl-ethyl-amino, n.-und i.-Propylamino und Methyl-n.- Butylamino; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl.
_{R}² für Wasserstoff steht,
_{R}³ für Wasserstoff steht,
R⁴ für Methyl, Ethyl die gegebenenfalls durch OH oder _{SCH3}, Phenyl, Hydroxyphenyl, COO Cₗ₋₄-Alkyl, CONH₂, Imidazolyl, Indolyl substituiert sein können, steht,
R⁵ für OH, C₁₋₄-Alkoxy, insbesondere Methoxy, Ethoxy; Amino, Monoalkylamino, insbesondere Methyl-, Ethyl-amino steht,
X für O, S steht.

Im einzelnen seien neben den in den Beispielen genannten Verbindungen die folgenden substituierten Harnstoffe der Formel Ia genannt:

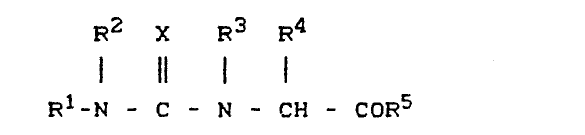

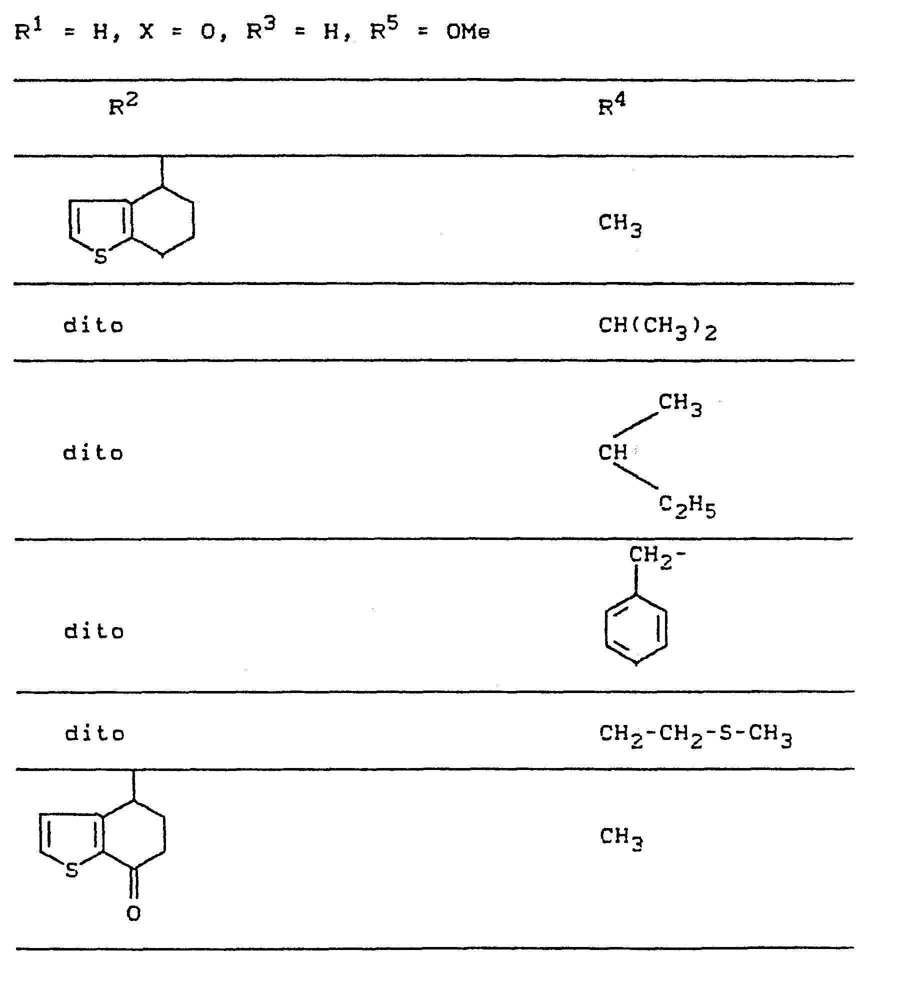

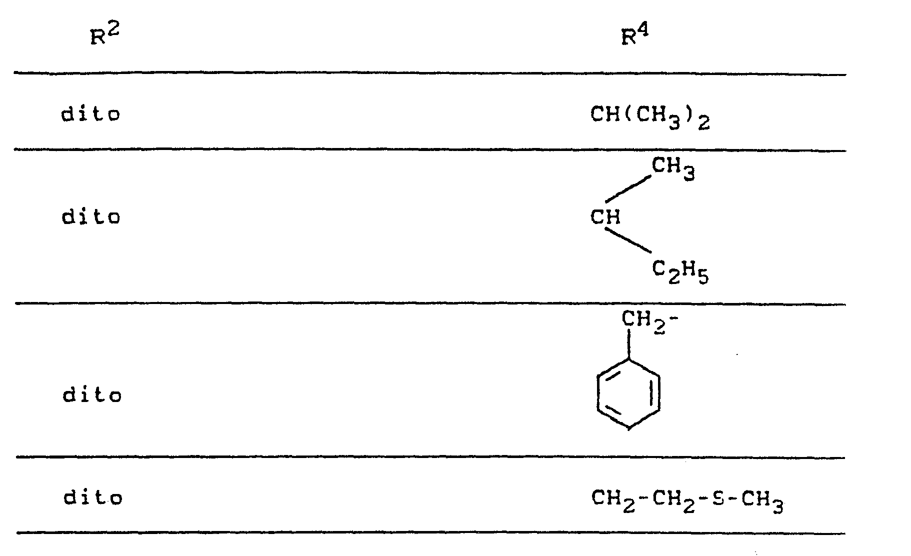

Im einzelnen seien neben den in den Beispielen genannten Verbindungen die folgenden substituierten Isoharnstoffe der Formel Ib genannt:

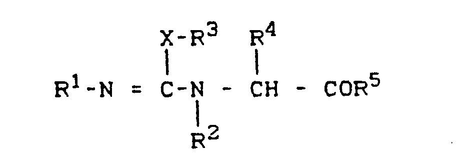

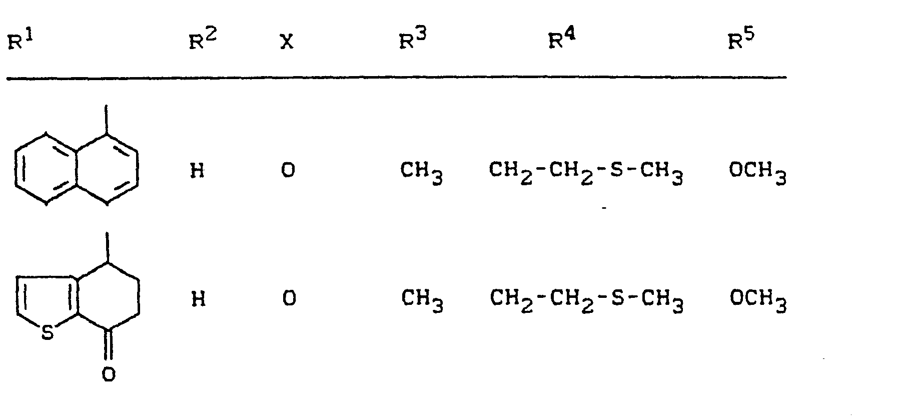

Von den neuen substituierten Harnstoffen sind bevorzugt diejenigen der Formel Ia in welcher
R¹ für C₁₋₄-Alkyl, ein- oder mehrcyclisches C₅-C₁₀-Cycloalkyl, C₅-C₇-Cycloalkanon, C₂-C₆-Alkenyl, C₆-C₁₀-Cycloalkenyl, C₅-C₇-Cycloalkenon, Naphthyl, Thiophen steht, die gegebenenfalls substituiert sein können,
R² für Wasserstoff oder C₁-C₄-Alkyl steht,
R³ für Wasserstoff oder C₁-C₄-Alkyl steht,
R⁴ für substituiertes C₁-C₃-Alkyl steht,
R⁵ für C₁-C₄-Alkyl, C₁-C₄-Alkoxy, Aryl, Aryloxy, die gegebenenfalls substituiert sein können, für Amino oder -NR⁶R⁷ steht,
R⁶ für Wasserstoff oder C₁-C₄-Alkyl steht,
R⁷ für Wasserstoff, C₁-C₄-Alkyl, C₂-C₄-Alkenyl, C₂-C₄-Alkinyl, Aryl, C₁-C₄-Aralkyl, die gegebenenfalls substituiert sein können, steht,
X für 0 oder S steht.

Von den neuen substituierten Isoharnstoffen sind bevorzugt diejenigen der Formel Ib, in welcher die Reste R¹, R², R³, R⁴, X, die bei den neuen substituierten Harnstoffen der Formel la als bevorzugt angegebenen Bedeutungen haben und der Rest R⁵ für Cₗ-C₄-Alkyl, Cₗ-C₄-Alkoxy, Aryl, Aryloxy, die gegebenenfalls substituiert sein können, steht.

Bevorzugt seien folgende Säuren genannt, die mit den Wirkstoffen der Formel I Salze bilden können:
HC1, H₂S0₄, HSO₄-, H₃PO₄, H₂PO₄-, HClO₄, HBr, HJ, HF, HNO₃, H₂CO₃, HCO₃⁻, H₃B0₃, HN₃,

Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfosäure, Nikotinsäure, Citronensäure, Ascorbinsäure.

Für den Fall R⁵ = OH seien folgende Basen genannt, die mit den Wirkstoffen der Formel 1 Salze bilden können:
NaOH, KOH, Alkali- und Erdalkalicarbonate, organische Base wie z.B. Triethylamin, Mono- und Dialkylamine, quartäre Ammoniumhydroxide.

Die bei 4 (oben) angegebenen Verfahren zur Herstellung der neuen substituierten Harnstoffe der Formel Ia werden durchgeführt, indem man die entsprechenden Amine der Formeln III oder IV mit den entsprechenden Isocyanaten umsetzt. Setzt man z.B. Ethylisocyanat und Methioninethylester ein, kann der Reaktionsablauf durch folgendes Formelschema wiedergegeben werden: 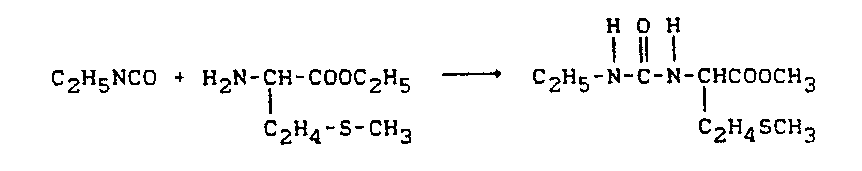 Die bei Verfahren 4a und b eingesetzten Isocyanate oder -thiocyanate der Formeln II und V sind bekannt oder lassen sich analog zu bekannten Methoden herstellen.

Bevorzugt werden Verbindungen der Formeln II und V eingesetzt, die zu den weiter oben als bevorzugt genannten neuen Wirkstoffen führen.

Im einzelnen seien Isocyanate oder -thiocyanate der Formel II genannt, die sich von folgenden Aminen ableiten:
Methylamin, Ethylamin, Propylamin, Isopropylamin, Butylamin, iso-Butylamin, tert.-Butylamin, Hexylamin, Dodecylamin, 2-Ethylhexylamin, Tetradecylamin, Hexadecylamin, Octadecylamin, 3-Butoxypropylamin, 3-Aminopropansäure-2-methylpropylester, 6-Aminohexanitril, 1,1- Aminoundecansäureester, Cyclohexylamin, Trimethylcyclohexylamin, 2-Norbornylmethylamin, Anilin, o-, m-, p-Chloranilin, 2,3-2,4-, 2,5-, 2,6-Dichloranilin, 3,4-, 3,5-Dichloranilin, p-, o-Nitroanilin, m-, o-, p-Tolylamin, 3-Trifluormethylanilin, 3-Chlor-4-methylanilin, 4-Chlor-3-methylanilin, Benzylamin, Phenylcyclohexylamin, Naphthylamin, Adamantylamin, außerdem 2-Amino-3-carbethoxythiophen, 3-Amino-2-carbethoxythiophen, 2-Amino-3-carbethoxy-4,5,6,7-tetrahydrobenzothiophen, 2-Amino-3-carbethoxy-4,5-di- methylthiophen, 2-Amino-3-carbethoxy-4-methyl-5-phenyl- thiophen.

Im einzelnen seien folgende Isocyanate oder -thiocyanate der Formel V genannt: (hergestellt nach P. Stelzel in Methoden der organ. Chemie (Houben-Weyl-Müller) Band XV/2, S. 183, Georg Thieme Verlag Stuttgart).
2-Isocyanatopropionsäuremethylester
2-Isocyanato-3-methylbuttersäuremethylesster
2-Isocyanato-4-methyl-valeriansäuremethylester
2-Isocyanato-3-phenyl-propionsäuremethylester
2-Isocyanato-3-methyl-pentansäure-methylester,

Die bei Verfahren 4a und b eingesetzten Amine bzw. Aminosäurederivate der Formeln III und IV sind bekannt oder lassen sich analog zu bekannten Methoden herstellen.

Bevorzugt werden Verbindungen der Formeln III und IV eingesetzt, die zu den weiter oben als bevorzugt genannten neuen Wirkstoffen führen.

Als Amine der Formel IV seien die weiter oben aufgeführten Amine genannt.

Die Verfahren 4a und 4b werden gegebenenfalls in Gegenwart von Verdünnungsmitteln und von Katalysatoren durchgeführt.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid und Pyridin.

Als Katalysatoren kommen die bei Umsetzungen mit Isocyanaten üblichen Katalysatoren infrage. Als solche seien genannt: tert.-Amine wie Triethylamin, N-Methylmorpholin, 1,4-Diaza-bicyclo-(2,2,2)-octan (DABCO) β,β'-Dimethyl- aminodiethylether, Dimethylbenzylamin, Metallkatalysatoren des Zn, Sn, Pb wie Dibutylzinndilaurat, Dibutylzinndioxid, Zinnoctoat, Bleioctoat, Zinkoctoat, Zinkchlorid, Zinkacetat, 4-Dimethylaminopyridin.

Die Reaktion wird zwischen 50 und 150°C, bevorzugt zwischen 60-110°C durchgeführt. Man arbeitet vorzugsweise unter Normaldruck.

Die Verbindungen der Formeln II und III bzw. IV und V werden in äquimolaren Mengen eingesetzt, ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase und Abdestillieren des Lösungsmittels.

Isoharnstoffe der Formel Ib lassen sich aus den entsprechenden Aminosäurederivaten der Formel VII durch Umsetzung mit den entsprechenden Imidokohlensäureesterhalogeniden der Formel VI herstellen. Verwendet man 1-Naphthyl-imidokohlensäureethylesterchlorid und Methioninmethylester, läßt sich der Reaktionsablauf durch das folgende Reaktionsschema wiedergeben: 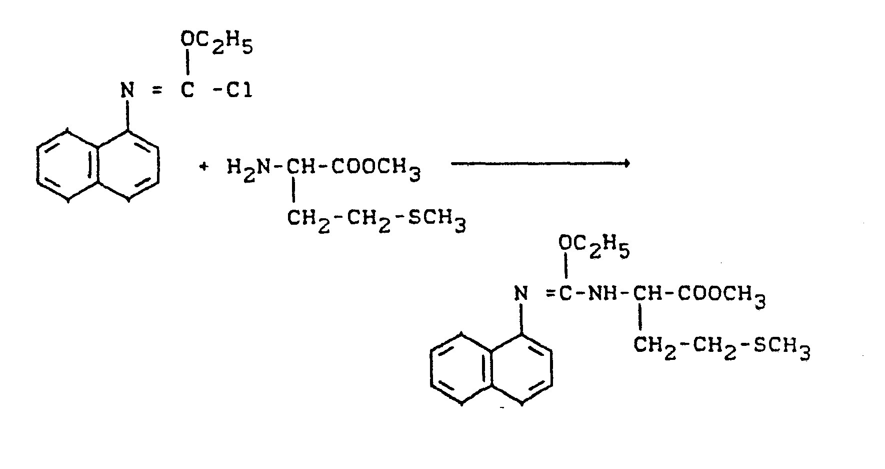 Es werden bevorzugt die Aminasäurederivate der Formel VII eingesetzt, die zu den weiter oben genannten bevorzugten Verbindungen der Formel Ib führen.

Imidokohlensäureesterhalogenide sind bekannt oder lassen sich analog zu bekannten Methoden herstellen. Bevorzugt werden Verbindungen der Formel VI eingesetzt, die zu den weiter oben als bevorzugt genannten neuen Wirkstoffen führen.

Halogen steht insbesondere für Chlor.

Im einzelnen seien folgende Imidokohlensäureesterhalogenide der Formel IV genannt:.

Ethyliminokohlensäuremethylesterchlorid Cyclohexyliminokohlensäureethylestserchlorid 1-Naphthyliminokohlensäuremethylesterchlorid Herstellung: E.Kühle in Methoden der Organischen Chemie (Houben-Weyl-Müller) Bd. E4, S. 544, Thieme Verlag, Stuttgart 1983.

Die Umsetzung erfolgt gegebenenfalls in Gegenwart von Säureakzeptoren, Katalysatoren und Verdünnungsmitteln.

Die Verbindungen der Formel VI und VII werden bevorzugt äquimolar eingesetzt. Ein Überschuß der einen oder anderen Komponente bringt keinen wesentlichen Vorteil.

Als Verdünnungsmittel kommen alle inerten orgnischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid.

Als Säureakzeptoren können alle üblichen Säurebindemittel verwendet werden. Hierzu gehören vorzugsweise Alkalicarbonate, -hydroxide oder -alkoholate, wie Natrium- oder Kaliumcarbonat, Natrium- und Kaliumhydroxid, Natrium- und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin und Pyridin.

Als Katalysatoren können Verbindungen verwendet werden, welche gewöhnlich bei Reaktionen in Zweiphasensystemen aus Wasser und mit Wasser nicht mischbaren organischen Lösungsmitteln zum Phasentransfer von Reaktanden dienen (Phasentransferkatalysatoren). Als solche sind vor allem Tetraalkyl- und Trialkylaralkyl-ammoniumsalze mit vorzugweise 1 bis 10, insbesondere 1 bis 8 Kohlenstoffen je Alkylgruppe, vorzugsweise Phenyl als Arylbestandteil der Aralkylgruppe und vorzugweise 1 bis 4, insbesondere 1 oder 2 Kohlenstoffatomen im Alkylteil der Aralkylgruppen bevorzugt. Hierbei sind vor allem die Halogenide, wie Chloride, Bromide und Iodide, mit vorzugsweise 1 bis 4, insbesondere 1 oder 2 Kohlenstoffatomen im Alkylteil der Aralkylgruppen bevorzugt. Vorzugsweise kommen die Chloride und Bromide infrage. Beispielhaft seien Tetrabutylammoniumbromid, Benzyl-triethylammoniumchlorid und Methyltrioctylammoniumchlorid genannt.

Die Reaktionstemperatur wird zwischen etwa 0°C und 130°C, vorzugsweise zwischen etwa 20°C und 60°C gehalten. Das Verfahren wird vorzugsweise bei Normaldruck durchgeführt.

Die Aufarbeitung erfolgt in üblicher Weise.

Die Wirkstoffe werden bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild; Pelztiere wie Nerze, Chinchilla; Geflügel wie z.B. Hühner, Gänse, Enten, Truthähne, Tauben; Fische wie z.B. Karpfen, Forellen, Lachse, Aale, Schleien, Hechte; Reptilien wie z.B. Schlangen und Krokodile.

Zu den Zier- und Hobbytieren zählen Säugetiere wie Hunde und Katzen; Vögel wie Papageien, Kanarienvögel; Fische wie Zier- und Aquarienfische z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Wachstums- und Leistungsphase, dem Gesundheitszustand und der Art der Haltung und Fütterung der Tiere ab und sind durch jeden Fachmann leicht zu ermitteln.

Die Wirkstoffe werden den Tieren nach den Üblichen Methoden verabreicht. Die Art der Verabreichung hängt insbesondere von der Art, dem Verhalten und dem Gesundheitszustand der Tiere ab.

Die Wirkstoffe können einmalig verarbeitet werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein-oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind:
z.B. Antibiotika wie Tylosin und Virginianycin. Mineralische Futtermittel sind z.B. Dicalciumphosphat, Magnesiumoxid, Natriumchlorid.

Spurenelemente-Verbindungen sind z.B. Eisenfumarat, Natriumjodid, Kobaltchlorid, Kupfersulfat, Zinkoxid. Vitamie sind z.B. Vitamin A, Vitamin D₃, Vitamin E, B-Vitamine, Vitamin C.

Nicht-Protein-Verbindungen sind z.B. Biuret, Harnstoff. Farbstoffe sind z.B. Carotinoide wie Citranaxanthin, Zeaxanthin, Capsanthin.

Antioxidantien sind z.B. Aethoxyquin, Butylhydroxy-toluol.

Aromastoffe sind z.B. Vanillin.

Emulgatoren sind z.B. Ester der Milchsäure, Lecithin. Fließhilfsstoffe sind z.B. Natriumstearat, Calciumstearat.

Konservierungsstoffe sind z.B. Zitronensäure, Propionsäure.

Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Er^{g}än- zungs-, Fertig- und Mischfuttermittel. Diese enthalten Einzelfuttermittel in einer Zusammensetzung, die eine ausgewogene Ernährung hinsichtlich der Energie- und Proteinversorgung sowie der Versorung mit Vitaminen, Mineralsalzen und Spurenelementen sicherstellen.

Die Konzentration der Wirkstoffe im Futter beträgt normalerweise etwa 0,01-500 ppm, bevorzuzgt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:
200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:
600 I.E. Vitamin A, 100 I.E. Vitamin D₃, 10 mg Vitamin E, 1 mg Vitamin K₃, 3 mg Riboflavin, 2 mg Pyridoxin, 20 mcg Vitamin B₁₂, 5 mg Calciumpantothenat, 30 mg Nikotinsäure, 200 mg Cholinchlorid, 200 mg Mn S0₂ x H₂0, 140 mg Zn S0₄ x 7 H₂0, 100 mg Fe S0₄ x 7 H₂0 und 20 mg Cu S0₄ x 5 N₂O.
2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält:
630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

### Beispiel A

### Ratten-Fütterungsversuch

Weibliche Laborratten 90-110 g schwer vom Typ SPF Wistar (Züchtung Hagemann) werden ad lib mit Standard-Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

### Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Gewichtszunahme und Futterverbrauch werden während des 13-tägigen Versuches bestimmt.

Bei Verwendung der Wirkstoffe der folgenden Beispiele werden deutliche Gewichtszunahmen im Vergleich zur Kontrolle erzielt: 1, 2, 3.

### Beispiel 1

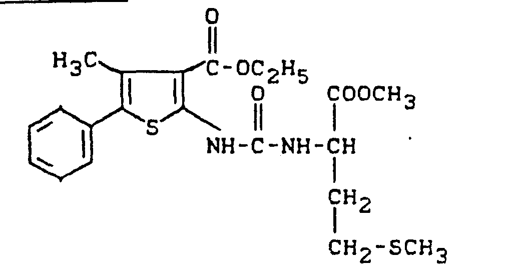

Zu 6,78 g (34 mmol) L-Methioninmethylesterhydrochlorid in 30 ml trockenem Choroform werden 3,4 g (34 mmol) Triethylamin zugegeben und noch 10 Minuten gerührt. Dann werden 6,5 g (22,5 mmol) 3-Carbethoxy-2-isocyanato-4-methyl-5-phenylthiophen, gelöst in 30 ml trockenem Chloroform zugetropft. Nach 30 Minuten ist die Reaktion beendet. Der Ansatz wird auf 300 ml Wasser gegossen, 200 ml Methylenchlorid zugegeben und die organische Phase abgetrennt. Die wäßrige Phase wird nochmals mit 150 ml Methylenchlorid nachextrahiert.

Die organische Phasen werden vereinigt und nacheinander mit 200 ml verdünnter Schwefelsäure, 200 ml Wasser und 200 ml NaHC0₃-Lösung gewaschen. Nach Trocknung mit Na₂S0₄ wird das Lösungsmittel unter vermindertem Druck abdestilliert und das Produkt durch Säulenchromatographie an Kieselgel mit Methylenchlorid/Essigester als Laufmittel gereinigt. Ausbeute 7 g (68,7 % der Theorie) eines gelblichen Öls.

IR: 3450, 3000, 1740, 1660, 1550, 1530 cm⁻¹.

### Beispiel 2

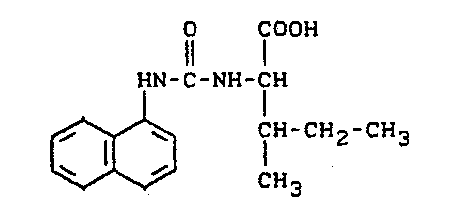

Zu 6,6 g (50 mmol) L-Isoleucin in 50 ml Wasser werden 2,7 g (25 mmol) Na₂C0₃ gegeben. Zu der entstandenen Lösung wird dann langsam eine Lösung von 8,5 g (50 mmol) 1-Naphthylisocyanat in 10 ml Dioxan zugetropft. Nach beendetem Zutropfen wird noch zwei Stunden nachgerührt, dann abfiltriert und das Produkt durch Ansäuren des Filtrats mit Ameisensäure gefällt. Nach Absaugen und Trocknung über KOH werden 6,9 g (46 % der Theorie) eines feinen Pulvers vom Schmelzpunkt 171°C (Zersetzung) erhalten.

### Beispiel 3

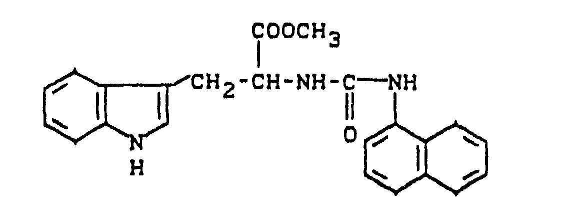

2,54 g (10 mmol) L-Tryptophanmethylesterhydrochlorid werden in 40 ml trockenem Chloroform suspendiert und 1,01 g (10 mmol) Triethylamin zugesetzt. Anschließend wird eine Lösung von 1,69 g (10 mmol) 1-Naphthylisocyanat bei Raumtemperatur zugetropft und noch 30 Minuten nachgerührt. Danach wird die Reaktionsmischung in 250 ml Wasser gegossen, die organische Phase abgetrennt und die wäßrige Phase nochmals mit 100 ml Chloroform nachextrahiert. Die vereinigten organischen Phasen werden dreimal mit je 100 ml Wasser gewaschen, dann mit Na₂S0₄ getrocknet und das Lösungsmittel im Vakuum abgedampft. Der Rückstand wird aus Toluol/Petrolether umkristallisiert. Ausbeute 2,5 g (64,6 % der Theorie) Schmelzpunkt 188°C.

### Beispiel 4

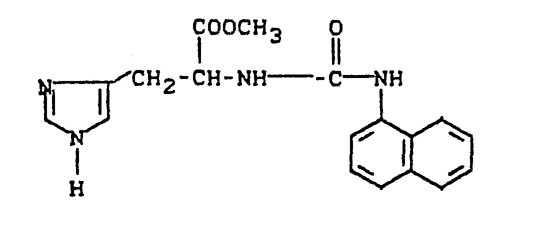

Zu einer Suspension von 5 g (20,7 mmol) L-Histidinmethylesterdihydrochlorid in 50 ml trockenem Chloroform werden 4,2 g (41,4 mmol) Triethylamin zugegeben und anschließend bei Raumtemperatur eine Lösung von 2,8 g (16,6 mmol) 1-Naphthylisocyanat in 20 ml trockenem Chloroform zugetropft. Es wird noch 30 Minuten bei Raumtemperatur gerührt, danach die Mischung auf 200 ml Wasser gegossen und das ausgefallene Produkt abgesaugt. Zur Reinigung wird in Ethanol gelöst und mit Wasser ausgefällt. Ausbeute 4,8 g (95 % der Theorie) Schmelzpunkt 132°C (Zersetzung).

Analog werden die foglenden Verbindungen hergestellt:

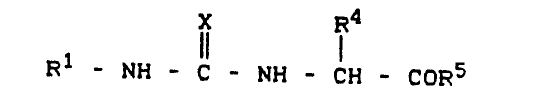

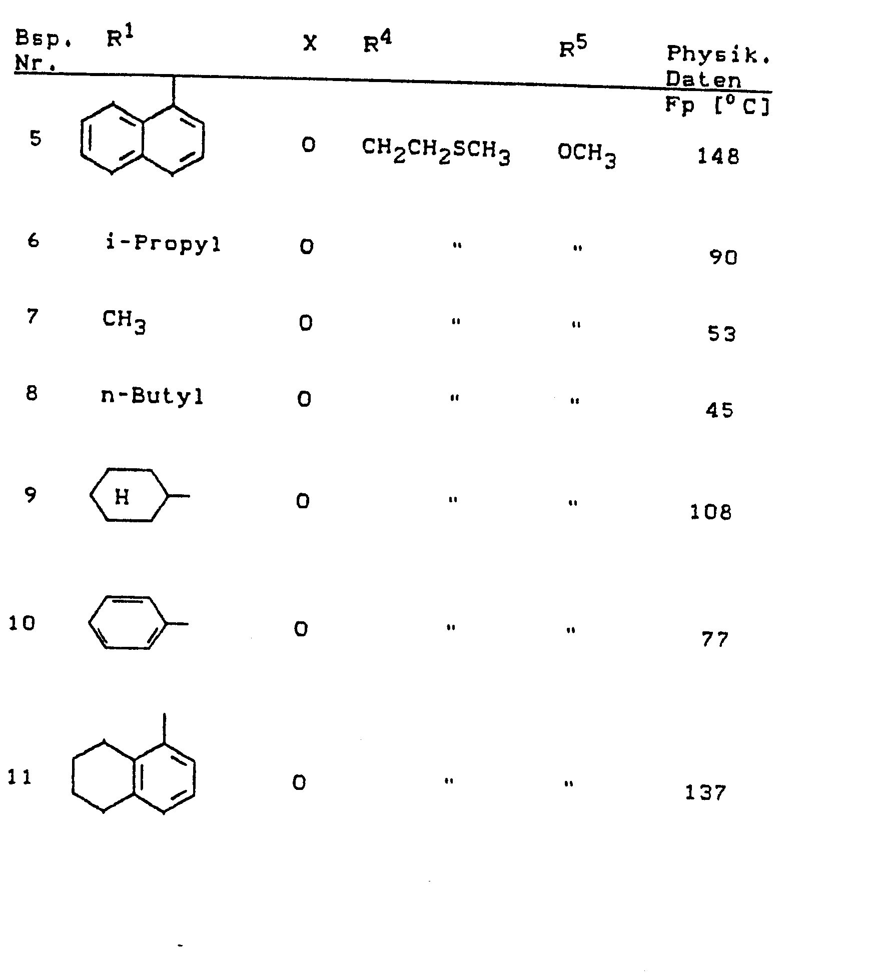

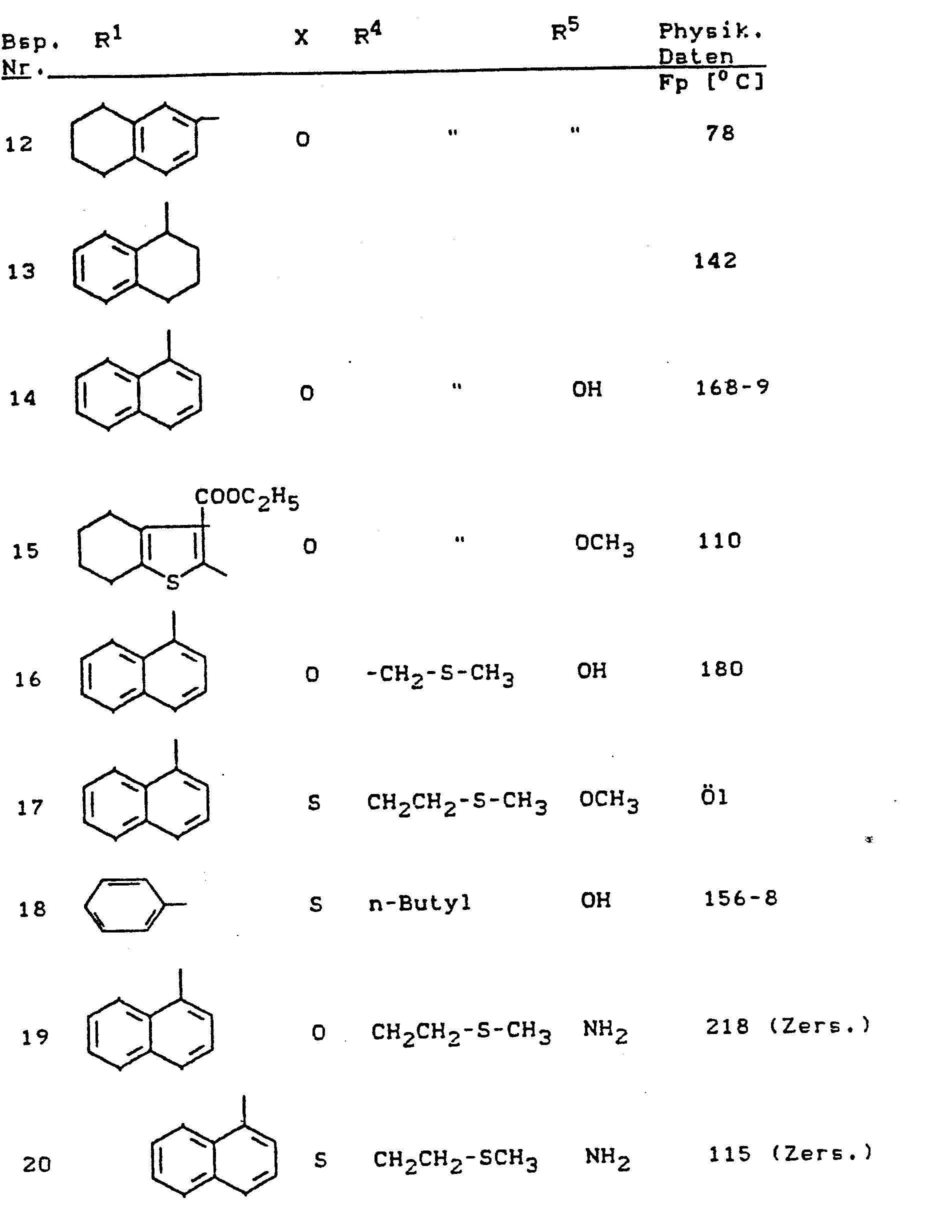

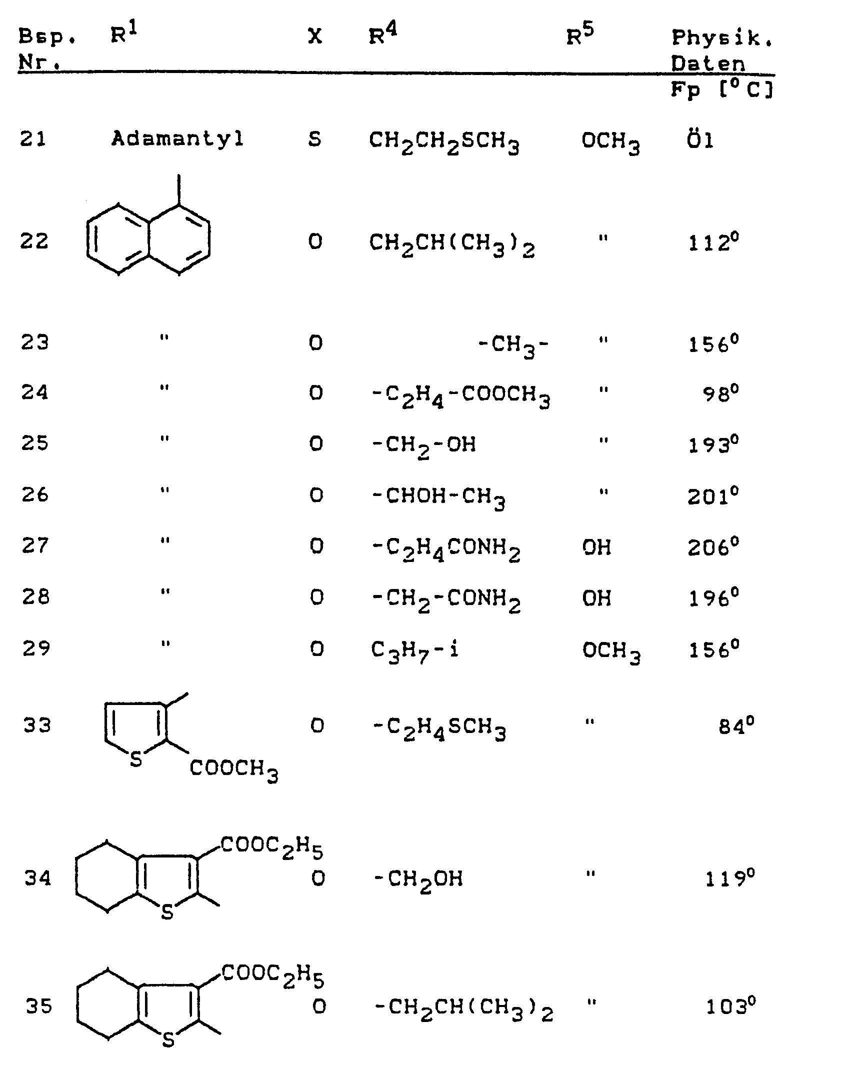

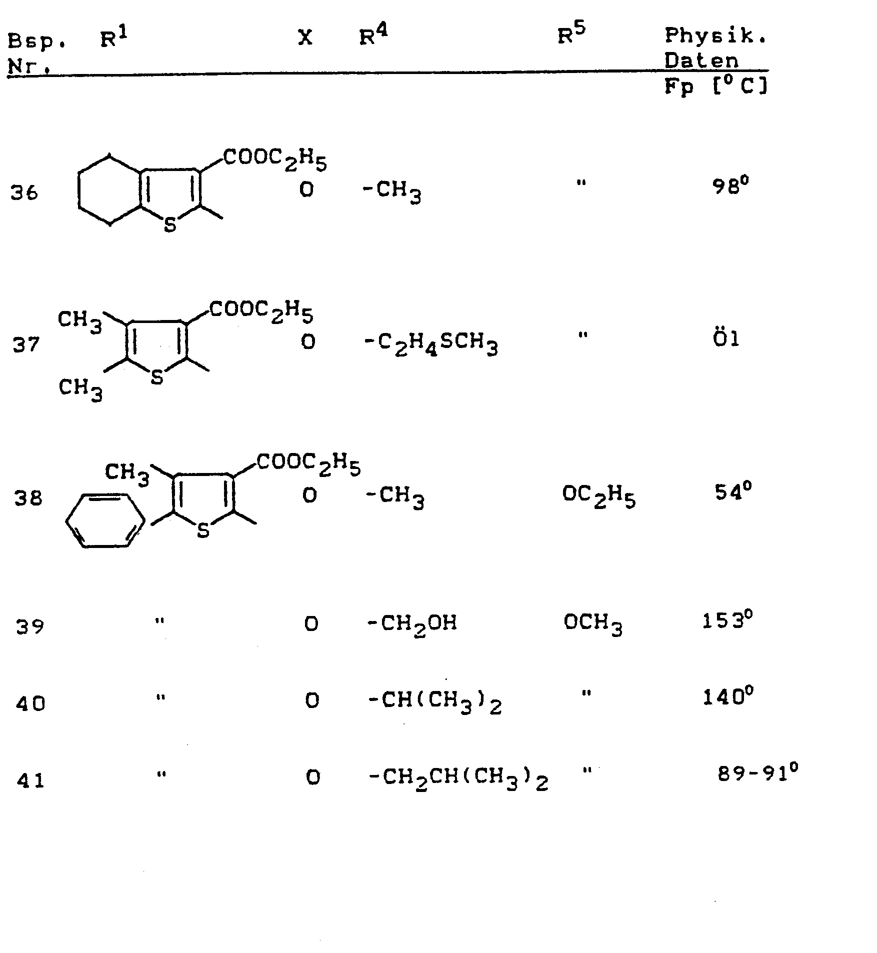

## Patentansprüche

1. Verwendung der substituierten Harnstoffe und Isoharnstoffe der Formeln Ia und Ib 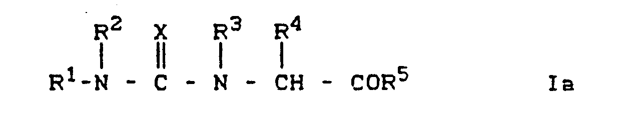 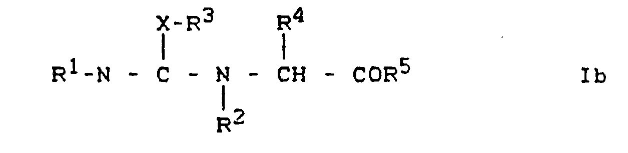 in welchen
R¹ Alkyl, ein- oder mehrcyclisches Cycloalkyl, Cycloalkanon, Aryl, Heteroaryl, Alkenyl, Cycloalkenyl, Cyloalkenoxy, die gegebenenfalls substituiert sein können steht,
R² für Wasserstoff oder Alkyl steht,
R³ für Wasserstoff oder Alkyl steht,
R⁴ für Alkyl steht, das gegebenenfalls substituiert sein kann,
_{R}³ und R⁴ können gemeinsam mit den Atomen, an die sie gebunden sind, einen gegebenenfalls susbtituierten 5-gliedrigen gesättigten Ring bilden,
_{R}⁵ für OH, Alkyl, Alkoxy, Aryl, Aryloxy, die gegebenenfalls substituiert sein können, Amino, -_{NR}⁶R⁷ steht,
_{R}⁶ für Wasserstoff oder Alkyl steht,
R⁷ für Wasserstoff, Alkyl, Alkenyl, Alkinyl, Aryl, Aralkyl, die gegebenenfalls substituiert sein können, steht,
X für 0 oder S steht,
als leistungsfördernde Mittel bei Tieren.
Die Wirkstoffe der Formeln Ia und Ib können dabei in Form ihrer Enantiomeren sowie in Form ihrer physiologisch verträglichen Salze vorliegen.

2. Substituierte Harnstoffe der Formel Ia 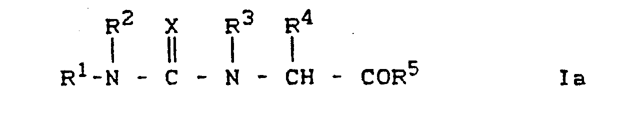 in welcher
_{R}¹ für Alkyl, ein- oder mehrcyclisches Cycloalkyl, Cycloalkanon, Alkenyl, Cycloalkenyl, Cycloalkenon, Naphthyl, Thiophen steht, die gegebenenfalls substituiert sein können,
R² für Wasserstoff oder Alkyl steht,
_{R}³ für Wasserstoff oder Alkyl steht,
R⁴ für substituiertes Alkyl steht,
R⁵ für Alkyl, Alkoxy, Aryl, Aryloxy, die gegebenenfalls substituiert sein können, für Amino oder -NR⁶R⁷ steht,
_{R}⁶ für Wasserstoff oder Alkyl steht,
R⁷ für Wasserstoff, Alkyl, Alkenyl, Alkinyl, Aryl, Aralkyl, die gegebenenfalls substituiert sein können, steht,
X für 0 oder S steht.

3. Substituierte Isoharnstoffe der Formel Ib 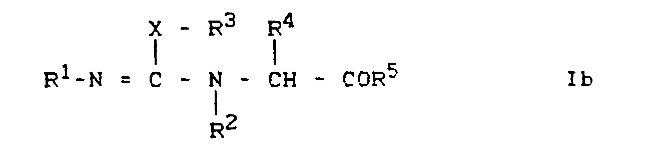 in welcher
R¹ für Alkyl, ein- oder mehrcyclisches Cycloalkyl, Cycloalkanon, Alkenyl, Cycloalkenyl, Cycloalkenon, Naphthyl, Thiophen steht, die gegebenenfalls substituiert sein können,
_{R}² für Wasserstoff oder Alkyl steht,
_{R}³ für Wasserstoff oder Alkyl steht,
R⁴ für substituiertes Alkyl steht,
R⁵ für Alkyl, Alkoxy, Aryl, Aryloxy, die gegebenenfalls substituiert sein können, für Amino oder -NR⁶R⁷ steht,
_{R}⁶ für Wasserstoff oder Alkyl steht,
R⁷ für Wasserstoff, Alkyl, Alkenyl, Alkinyl, Aryl, Aralkyl die gegebenenfalls substituiert sein können steht,
X für 0 oder S steht.

4. Verfahren zur Herstellung substituierter Harnstoffe der Formel Ia 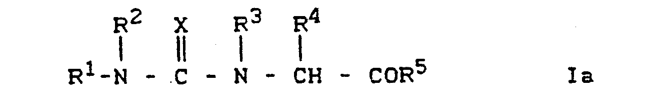 in welcher
R¹ für Alkyl, ein- oder mehrcyclisches Cycloalkyl, Cycloalkanon, Alkenyl, Cycloalkenyl, Cycloalkenon, Naphthyl, Thiophen steht, die gegebenenfalls substituiert sein können,
_{R}² für Wasserstoff oder Alkyl steht,
R³ für Wasserstoff oder Alkyl steht,
R⁴ für substituiertes Alkyl steht,
_{R}⁵ für Alkyl, Alkoxy, Aryl, Aryloxy, die gegebenenfalls substituiert sein können, für Amino oder -NR⁶R⁷ steht,
R⁶ für Wasserstoff oder Alkyl steht,
R⁷ für Wasserstoff, Alkyl, Alkenyl, Alkinyl, Aryl, Aralkyl, die gegebenenfalls substituiert sein können, steht,
X für O oder S steht,
a) für den Fall, daß R² für Wasserstoff steht, Isocyanate oder -thiocyanate der Formel II 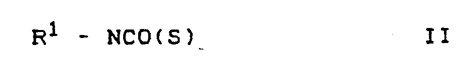 in welcher
R¹ die oben angegebene Bedeutung hat, mit Aminosäurederivaten der Formel III 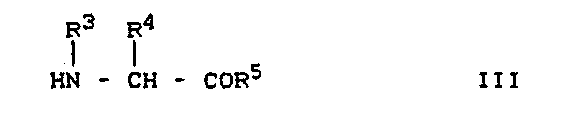
in welcher
_{R}³, _{R}⁴, R⁵ die oben angegene Bedeutung haben,
gegebenenfalls in Gegenwart von Katalysatoren und Verdünnungsmitteln umetzt, oder
b) für den Fall, daß R³ für Wasserstoff steht, Amine der Formel IV 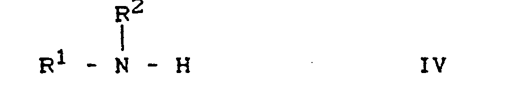 in welcher
R¹ und R² die oben angegebene Bedeutung haben, mit Isocyanaten oder -thiocyanaten der Formel V 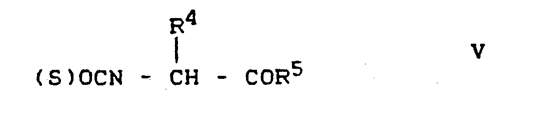 in welcher
R⁴ und R⁵ die oben angegebene Bedeutung haben, gegebenenfalls in Gegenwart von Katalysatoren und Verdünnungsmitteln umsetzt.

5. Verfahren zur Herstellung von substituierten Isoharnstoffen der Formel Ib 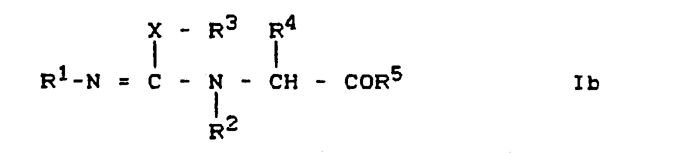 in welcher
R¹. _{R}², _{R}³, R⁴, _{R}⁵, _{X} die in Anspruch 3 angegebene Bedeutung haben,
dadurch gekennzeichnet, daß man Imidokohlensäureesterhalogenide der Formel VI 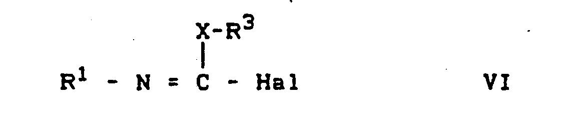 in welcher
R¹ und R³ und X die oben angegebene Bedeutung haben und
Hal für Halogen steht,
mit Aminosäurederivaten der Formel VII 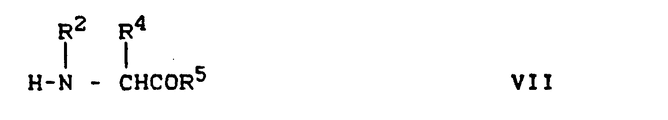 in welcher
_{R}², R4, R⁵ die oben angegebene Bedeutung haben,
umsetzt.

6. Wachstumsfördernde Mittel für Tiere, gekennzeichnet durch einen Gehalt an substituierten Harnstoffen und Isoharnstoffen der Formeln Ia und Ib gemäß Anspruch 1.

7. Futter und Futterzusatzmittel für Tiere, gekennzeichnet durch einen Gehalt an substituierten Harnstoffen und Isoharnstoffen der Formeln Ia und Ib gemäß Anspruch 1.

8. Verfahren zur Herstellung wachstumsfördernder Mittel, Futter und Futterzusatzmittel für Tiere, dadurch gekennzeichnet, daß man substituierte Harnstoffe und Isoharnstoffe der Formeln Ia und Ib gemäß Anspruch 1 und Träger- und Hilfsstoffen vermischt.
